# EUROPÄISCHE PATENTSCHRIFT

(11) **EP 4 032 576 B1**
(45) Veröffentlichungstag und Bekanntmachung des Hinweises auf die Patenterteilung: **18.11.2026**
(21) Anmeldenummer: 21152560.5
(22) Anmeldetag: 20.01.2021
(51) Int. Cl.: A61M 25/00

(54) **KATHETER MIT KATHETERSPITZENELEMENT**
CATHETER WITH CATHETER TIP ELEMENT
CATHÉTER AVEC ÉLÉMENT DE POINTE DE CATHÉTER

(43) Veröffentlichungstag der Anmeldung: 27.07.2022
(73) Patentinhaber: Hörning, Heike, 60314 Frankfurt (DE)
(72) Erfinder: HÖRNING, Heike, 60314 Frankfurt (DE); REUTER, Jürgen, 36211 Alheim (DE)
(74) Vertreter: Prüfer & Partner mbB Patentanwälte · Rechtsanwälte

(56) Entgegenhaltungen:
- WO-A2-2010/090671
- US-A1- 2009 030 370
- US-A1- 2015 018 802
- US-A1- 2019 224 455
- US-B1- 10 010 392

## Beschreibung

Die Erfindung betrifft einen Katheter mit einem Katheterspitzenelement. Katheter werden vielfach zur Einführung in Hohlorgane, wie Harnblase, Magen, Darm, Gefäße oder Herz, verwendet, um diese zu sondieren, zu entleeren, zu füllen und/oder zu spülen. Beispielsweise wird Patienten, bei denen eine Entleerung der Harnblase nicht mehr auf natürliche Weise erfolgt, ein Blasenkatheter gelegt. Solche Blasenkatheter werden im Allgemeinen von außen durch die Harnröhre bis in die Harnblase geschoben (transurethraler Blasenkatheter) und können dort 4 bis 6 Wochen verbleiben. Zur leichteren Einführung transurethraler Katheter durch die Harnröhre und durch weitere Organe, wie die Prostata, weisen diese in einigen Ausführungsformen eine leicht abgewinkelte, flexible Spitze, auch als Tiemann-Spitze bezeichnet, auf.

Bei einem längeren Verbleib des Blasenkatheters in der Harnblase neigt diese zur Schrumpfung aufgrund der steten Harnableitung. Eine solche Schrumpfung kann bedingen, dass die Katheterspitze, wie die vorstehende Tiemann-Spitze, an der Innenwand der Harnblase, insbesondere immer wieder an der gleichen Stelle, anliegt und dort reibt. Auch wenn die vorstehend beispielhaft genannte Katheterspitze eine gewisse Flexibilität aufweist, ist die dennoch für die Einführung erforderliche Steifigkeit ausreichend hoch, um Schleimhautreizungen bis hin zu Schleimhautblutungen an der Innenwand der Harnblase verursachen zu können. Dies kann nicht nur in Schmerzen für den Patienten resultieren, sondern auch chronische Verletzungen der gut durchbluteten und sensiblen Blasenschleimhaut und somit eine Narbenbildung bedingen. Das gegenüber einer gesunden Schleimhaut instabilere Narbengewebe kann seinerseits wiederum erneut der Ursprung von Schleimhautblutungen werden.

Zudem besteht das Risiko, dass bei einer Verletzung der Blasenschleimhaut Harnflüssigkeit in den Blutkreislauf gelangen kann, was wiederum die Gefahr einer Sepsis bedingt.

Zur Vermeidung der vorstehenden Probleme oder auch aufgrund anderer Indikationen kann der Katheter auch durch die Bauchdecke in die Harnblase eingeführt werden (suprapubischer Blasenkatheter). Dieser bedarf hinsichtlich des alternativen Einführungswegs keiner vergleichsweise steifen Katheterspitze, die bei weiterem Verbleib entsprechende Reibungen verursacht. Die Verwendungsmöglichkeit suprapubischer Blasenkatheter ist jedoch aufgrund von Kontraindikationen und/oder auch mangelnder Akzeptanz des Patienten beschränkt.

Die US 2015/0018802 A1 betrifft einen Katheter, der einen mittleren Abschnitt aufweist, der ein längliches, im Wesentlichen röhrenförmiges Element umfasst, und einen distalen Abschnitt aufweist, der ein distales steifes Element umfasst. Es wird eine Katheterisierungsvorrichtung bereitgestellt, die den Katheter und einen länglichen Innenkatheter umfasst, der in den Katheter einführbar ist. Es wird ein Verfahren zur Katheterisierung bereitgestellt, umfassend das Bereitstellen des Katheters, das Versteifen eines Abschnitts des Katheters und das Einführen des versteiften Katheters in eine Körperleitung eines Patienten.

Die WO 2010/090671 A2 betrifft einen Foley-Katheter, der einen Katheterschlauch mit einem proximalen Drainageende und einem distalen Einführungsende und mit einem Urindrainagelumen umfasst, das sich entlang einer Längsachse erstreckt. Der Katheterschlauch hat eine Urinabflussöffnung an seinem proximalen Abflussende und eine Urinaufnahmeöffnung an seinem distalen Einführungsende. Die Urinabfluss- und Aufnahmeöffnungen definieren die gegenüberliegenden Enden des Urinabflusslumens, um dadurch den Urinfluss durch den Katheterschlauch von einem Ende zum anderen aufzunehmen. Ferner ist zumindest die Urinaufnahmeöffnung am distalen Einführungsende des Katheterschlauchs um die Längsachse des Urindrainagelumens herum angeordnet. Bei dieser Anordnung enthält der Foley-Katheter auch einen aufblasbaren und entleerbaren externen Ballon, der dem distalen Einführungsende des Katheterschlauchs zugeordnet ist, um das distale Einführungsende innerhalb der Blase zu halten.

Siehe weiterhin US 10 010 392 B1, US 2009/030370 A1 und US 2019/224455 A1.

In Anbetracht der vorgenannten Nachteile ist es somit eine Aufgabe der vorliegenden Erfindung, einen Katheter bereitzustellen, der die oben genannten Nachteile vermeidet und insbesondere das Risiko des Wundreibens durch die Katheterspitze in einem Hohlorgan reduzieren kann.

Die Aufgabe wird durch einen Katheter mit einem Katheterspitzenelement gemäß dem unabhängigen Anspruch 1 gelöst. Vorteilhafte Weiterbildungen der Erfindung sind in den abhängigen Ansprüchen enthalten.

Erfindungsgemäß umfasst ein Katheter einen flexiblen Katheterschlauch mit einer einführungsseitigen Öffnung an einem einführungsseitigen Ende des Katheterschlauchs und ein Katheterspitzenelement, das derart konfiguriert ist, dass das Katheterspitzenelement von einer ersten Konfiguration, in der das Katheterspitzenelement zumindest abschnittsweise aus oder von der einführungsseitigen Öffnung in eine dem Katheterschlauch abgewandten Richtung vorsteht und eine geeignete Steifigkeit für eine Einführung des Katheters aufweist, in eine zweite Konfiguration, in der ein durch das vorstehende Katheterspitzenelement gebildeter Überstand des Katheterspitzenelements eine geringere Steifigkeit als in der ersten Konfiguration aufweist, überführbar ist.

Das Katheterspitzenelement kann demnach als ein separates Element ausgebildet werden, das auf die einführungsseitige Öffnung des Katheterschlauchs aufgesetzt und/oder in diese eingesetzt wird, so dass das Katheterspitzenelement in der ersten Konfiguration zumindest abschnittsweise aus der einführungsseitigen Öffnung vorsteht. Alternativ kann das Katheterspitzenelement aber auch integral mit dem Katheterschlauch ausgebildet werden, wobei das Katheterspitzenelement von der einführungsseitigen Öffnung vorsteht. Das Katheterspitzenelement schließt sich somit an die einführungsseitige Öffnung der Katheterschlauchs an.

Der Begriff der geeigneten Steifigkeit des Katheterspitzenelements in der ersten Konfiguration zur Einführung, wie beispielsweise durch eine Harnröhre in eine Harnblase, bezieht sich dabei auf die Eigenschaft des Katheterspitzenelements in der ersten Konfiguration insgesamt. Die geeignete Steifigkeit kann dabei unter anderem durch das Material des Katheterspitzenelements selbst und/oder Vorspannungsmechanismen, wie durch die später noch beschriebene Beaufschlagung des Katheterspitzenelements mit einem positiven Innendruck, erreicht werden. Der Grad der Steifigkeit in Bezug auf seine Geeignetheit kann applikationsbedingt unterschiedlich sein. Beispielsweise wäre für eine Einführung in eine Harnblase eines männlichen Patienten aufgrund des durch die Prostata bestehenden Hindernisses auf dem Weg durch die Harnröhre eine andere geeignete Steifigkeit vorzusehen als für eine Einführung in ein Hohlorgan ohne erwartbare Hindernisse oder zumindest mit geringeren Widerständen.

In einer zweiten Konfiguration kann dann die Steifigkeit des Katheterspitzenelements gegenüber der Steifigkeit in der ersten Konfiguration verringert sein. Durch den reduzierten Überstand bis hin zu dessen Wegfall wird das Risiko minimiert, dass das Katheterspitzenelement überhaupt mit einer Innenwand eines Hohlorgans in Kontakt kommt. Durch die alternative oder ergänzende Verringerung der Steifigkeit des Katheterspitzenelements kann zudem eine unter Umständen immer noch mögliche Reibung in ihrer Auswirkung reduziert werden.

Der Grundgedanke der vorliegenden Erfindung beruht somit zusammengefasst darauf, dass ein im anwendungsgerechten Gebrauch im Hohlorgan verbleibendes Katheterende nur noch über die durch den Katheterschlauch einführungsseitige Öffnung oder ein für den Verbleib im Hohlorgan in seiner Steifigkeit im Hinblick auf ein Reibungsrisiko tolerierbares Katheterspitzenelement verfügt. Hierzu wird das Katheterspitzenelement erfindungsgemäß von der ersten Konfiguration in die zweite Konfiguration überführt.

Zudem ist in der zweiten Konfiguration der durch das vorstehende Katheterspitzenelement gebildete Überstand des Katheterspitzenelements gegenüber der einführungsseitigen Öffnung zumindest teilweise aufgehoben.

In der zweiten Konfiguration kann dann das Katheterspitzenelement zumindest einen geringeren, insbesondere im Wesentlichen keinen Überstand aus bzw. von der einführungsseitigen Öffnung des Katheterschlauchs mehr aufweisen. Somit verfügt ein im anwendungsgerechten Gebrauch im Hohlorgan verbleibendes Katheterende nur noch über die durch den Katheterschlauch einführungsseitige Öffnung oder ein für den Verbleib im Hohlorgan in seiner Abmessung im Hinblick auf ein Reibungsrisiko tolerierbares Katheterspitzenelement.

Das Katheterspitzenelement ist zur Überführung von der ersten Konfiguration in die zweite Konfiguration in einer Erstreckungsrichtung des Katheterschlauchs von der einführungsseitigen Öffnung zu einem der einführungsseitigen Öffnung abgewandten Ende hin zumindest abschnittsweise relativ zum Katheterschlauch bewegbar.

Durch die Relativbewegung zwischen dem Katheterspitzenelement und dem Katheterschlauch kann der Überstand des Katheterspitzenelements aus bzw. von der einführungsseitigen Öffnung teilweise oder vollständig aufgehoben werden. Dazu kann das Katheterspitzenelement beispielsweise durch die einführungsseitige Öffnung des Katheterschlauchs in der Erstreckungsrichtung des Katheterschlauchs in Richtung des der einführungsseitigen Öffnung abgewandten Endes in den Katheterschlauch hinein- bzw. zurückgezogen werden. Sofern das Katheterspitzenelement als vom Katheterschlauch separates Element ausgebildet ist, kann somit der Überstand korrespondierend zur Relativbewegung reduziert werden. Zudem kann das Katheterspitzenelement über eine entsprechende Relativbewegung auch vollständig aus dem Katheter entfernt werden, um zum Beispiel den Katheterschlauch nicht innenliegend zu blockieren oder um einen erneuten Überstand durch eine unbeabsichtigte entgegengesetzte Relativbewegung zu verhindern. Für eine vergleichbare Funktionsweise kann ein integral mit dem Katheterschlauch ausgebildetes Katheterspitzenelement auch eine Sollbruchstelle aufweisen, so dass ein Ablösen des Katheterspitzenelements durch die Initiierung der Relativbewegung über die Sollbruchstelle erfolgt und das abgelöste Katheterspitzenelement als separates Katheterspitzenelement analog zu den vorstehenden Ausführungen bewegt werden kann. In einer weiteren Variante kann auch nur ein Teil des Katheterspitzenelements relativ zum Katheterschlauch bewegt werden und nicht das Katheterspitzenelement insgesamt, wie dies später noch beschrieben wird.

Insbesondere weist das Katheterspitzenelement zumindest bei der Überführung von der ersten Konfiguration in die zweite Konfiguration einen maximalen Durchmesser senkrecht zur Erstreckungsrichtung des Katheterschlauchs auf, der kleiner oder gleich zumindest einem minimalen Innendurchmessers eines Rücknahmeabschnitts des Katheterschlauchs für das Katheterspitzenelement ist, um eine Rückzugsbewegung in den Katheterschlauch hinein zu ermöglichen.

Als Rücknahmeabschnitt des Katheterschlauchs wird der Bereich bezeichnet, der für eine Aufnahme und/oder weitere Rückführung des Katheterspitzenelements oder zumindest eines zur Reduzierung des Überstands vorbestimmten Abschnitts vorgesehen ist. Sofern das Katheterspitzenelement beispielsweise gemäß der vorstehend beschriebenen Variante vollständig aus dem Katheterschlauch herausgezogen wird, erstreckt sich der Rücknahmeabschnitt somit von der einführungsseitigen Öffnung des Katheterschlauchs zumindest bis zu dem Bereich des Katheterschlauchs, der für die Herausführung des Katheterspitzenelements in entgegengesetzter Richtung zur einführungsseitigen Öffnung vorgesehen ist. In einer einfachen Ausgestaltung des Katheterschlauchs weist dieser einen konstanten Innendurchmesser auf, der über die gesamte Länge des Katheterschlauchs einen Rücknahmeabschnitt ausbilden kann. Der Innendurchmesser des Rücknahmeabschnitts entspricht somit dem Innendurchmesser des Katheterschlauchs. Mit anderen Worten bildet der Katheterschlauch in diesem Fall den Rücknahmeabschnitt aus.

Zur Relativbewegung zumindest eines Teils des Katheterspitzenelements gegenüber dem Katheterschlauch bzw. dem Rücknahmeabschnitt zur Überführung des Katheterspitzenelements von der ersten Konfiguration in die zweite Konfiguration kann demnach das Durchmesserverhältnis des Katheterspitzenelements und des Rücknahmeabschnitts in einer Richtung senkrecht zur Erstreckungsrichtung des Katheterschlauchs so angepasst sein, dass dieses kleiner oder gleich 1 ist. Das Katheterspitzenelement kann hierzu an sich in der ersten Konfiguration schon einen maximalen äußeren Durchmesser zumindest in einem durch den Rücknahmeabschnitt aufzunehmenden Bereich aufweisen, der bei einer Überführung von der ersten in die zweite Konfiguration kleiner oder gleich dem minimalen inneren Durchmesser des Rücknahmeabschnitts ist, oder auf einen solchen maximalen äußeren Durchmesser anpassbar sein. Alternativ oder ergänzend kann aber auch der Rücknahmeabschnitt zur Überführung des Katheterspitzenelementabschnitts von der ersten Konfiguration in die zweite Konfiguration einen anpassbaren minimalen inneren Durchmesser aufweisen, um das erforderliche Durchmesserverhältnis für eine Relativbewegung vorsehen zu können. Beispielsweise könnte der Katheterschlauch als Rücknahmeabschnitt für die Überführung des Katheterspitzenelements von der ersten Konfiguration in die zweite Konfiguration mit einem Innendruck beschaufschlagt werden, um den Rücknahmeabschnitt aufzuweiten, also den Innendurchmesser zu vergrößern.

Gemäß einer Weiterbildung weist zumindest der in der ersten Konfiguration aus und/oder von der einführungsseitigen Öffnung vorstehende Abschnitt des Katheterspitzenelements zumindest einen verformbaren Abschnitt aufweist.

Demnach kann das Katheterspitzenelement zur Überführung von der ersten Konfiguration in die zweite Konfiguration verformt werden. Die Verformung kann sowohl elastisch, also reversibel, als auch plastisch, also irreversibel sein. Die Verformung kann dazu genutzt werden, das Katheterspitzenelement in seinem maximalen äußeren Durchmesser soweit zu reduzieren, dass dieses in Verbindung mit dem minimalen inneren Durchmesser des Rücknahmeabschnitts eine entsprechende Relativbewegung ausführen kann. Alternativ oder ergänzend kann die Verformung aber auch dazu genutzt werden, einen im Hohlorgan verbleibenden Überstand zu minimieren. Hierzu kann bevorzugt eine plastische Verformung genutzt werden.

Für eine Reduzierung des maximalen äußeren Durchmessers des Katheterspitzenelements bzw. des maximalen äußeren Durchmessers zumindest eines im Rücknahmeabschnitt aufzunehmenden Teils des Katheterspitzenelements kann das Katheterspitzenelement bzw. der entsprechende Teil davon beispielsweise als elastischer federnder Hohlkörper ausgebildet sein. Die Wandung des elastisch federnden Hohlkörpers kann durch die bei einer Initiierung der Relativbewegung des Katheterspitzenelements in den Rücknahmeabschnitt hinein, also bei einer Bewegungsinitiierung in Erstreckungsrichtung des Katheterschlauchs in eine einführungsseitigen Öffnung entgegengesetzte Richtung, nach innen gedrückt werden. Durch die federnde Ausgestaltung kann gleichzeitig in der ersten Konfiguration eine geeignete Steifigkeit für die Einführung in ein jeweiliges Hohlorgan erreicht werden. Alternativ kann die Katheterspitze analog hierzu auch als elastisch federnder Vollkörper ausgebildet sein, wobei sich hierbei die Gestaltungsmöglichkeiten im Wesentlichen auf die Eigenschaften der eingesetzten Materialien begrenzen. Weiterhin alternativ oder ergänzend kann das Katheterspitzenelement auch zumindest abschnittsweise eine federnde Beschichtung aufweisen, über die an sich oder in Kombination mit dem vorstehenden elastisch federnden Hohl- oder Vollkörper ebenfalls eine Komprimierung auf einen komprimierten maximalen äußeren Durchmesser des Katheterspitzenelements zur Überführung von der ersten Konfiguration in die zweite Konfiguration erreicht werden kann. Die federnde Beschichtung kann in Verbindung mit dem elastisch federnden Hohl- oder Vollkörper oder auch einem starren Grundkörper des Katheterspitzenelements insbesondere zur Beibehaltung einer geeigneten Steifigkeit in der ersten Konfiguration dienen, da die Verformung der Beschichtung ausreichend sein kann, um den elastisch federnden Hohl- oder Vollkörper nur noch geringfügig bzw. den starren Grundkörper gar nicht verformen zu müssen.

Insbesondere ist zumindest der zumindest eine verformbare Abschnitt des Katheterspitzenelements so konfiguriert, dass er mit einem positiven und/oder negativen Innendruck beaufschlagbar ist.

Der zumindest eine verformbare Abschnitt des Katheterspitzenelements muss demnach nicht selbst eine geeignete Steifigkeit zur Einführung in ein Hohlorgan in der ersten Konfiguration aufweisen, sondern kann die geeignete Steifigkeit durch eine Beaufschlagung mit einem positiven Innendruck erreichen. Entsprechend ist zumindest der zumindest eine verformbare Abschnitt als Hohlkörperabschnitt ausgebildet. Die Druckzuführung kann beispielsweise über den Katheterschlauch erfolgen.

Alternativ oder ergänzend kann der zumindest eine verformbare Abschnitt des Katheterspitzenelements auch mit einem negativen Innendruck beaufschlagt werden, um das Katheterspitzenelement bzw. den zumindest einen verformbaren Abschnitt zu komprimieren bzw. sein Volumen zu verkleinern. Hierdurch kann das Katheterspitzenelement für eine Relativbewegung zur Überführung von der ersten Konfiguration in die zweite Konfiguration zumindest in seinem maximalen äußeren Durchmesser reduziert und/oder ein im Hohlorgan verbleibender Überstand verkleinert werden.

Der positive und/oder negative Innendruck kann bedarfsgerecht anpassbar sein, um für verschiedene Applikationen und/oder Anwendungsschritte unterschiedliche Steifigkeiten und/oder Komprimierungen bzw. Volumenverkleinerungen bereitstellen zu können.

Gemäß einer Ausgestaltung ist der in der ersten Konfiguration aus und/oder von der einführungsseitigen Öffnung vorstehende Überstand des Katheterspitzenelements zumindest abschnittsweise in Richtung der Erstreckungsrichtung des Katheterschlauchs nach innen, insbesondere in den Katheterschlauch hinein, stülpbar.

Somit muss nicht das gesamte Katheterspitzenelement relativ zum Katheterschlauch bewegt werden, sondern ein der einführungsseitigen Öffnung zugewandtes Ende des Katheterspitzenelements kann relativ zur einführungsseitigen Öffnung ortsfest verbleiben. Zur Überführung des Katheterspitzenelements von der ersten Konfiguration in die zweite Konfiguration ist dann lediglich ein der einführungsseitigen Öffnung abgewandtes Ende und/oder ein zwischen dem der einführungsseitigen Öffnung abgewandten und zugewandten Ende befindlicher Bereich des Katheterspitzenelements nach innen in den Kathederschlauch durch die einführungsseitige Öffnung hinein bewegbar. Sofern ein restlicher verbleibender Überstand des Katheterspitzenelements im Hohlorgan tolerierbar ist, kann der Überstand auch in sich selbst in Richtung des Katheterschlauchs nach innen einstülpbar sein, ohne zwingend durch die einführungsseitige Öffnung in dem Katheterschlauch aufgenommen zu werden.

Erfindungsgemäß umfasst das Katheterspitzenelement ein flexibles Rücknahmeelement, das sich ausgehend von dem Katheterspitzenelement zumindest abschnittsweise durch den Katheterschlauch erstreckt und zumindest in der Erstreckungsrichtung des Katheterschlauchs relativ zu diesem bewegbar ist.

Über das Rücknahmeelement kann der aus oder von der einführungsseitigen Öffnung vorstehende Überstand des Katheterspitzenelements von der ersten in die zweite Konfiguration durch Aufbringen einer Zugkraft und somit Initiierung und Durchführung der Relativbewegung des Katheterspitzenelements in Bezug auf den Katheterschlauch bewegt werden. Das Rücknahmeelement wird dabei an einem der einführungsseitigen Öffnung gegenüberliegenden Ende des Katheterschlauchs oder an anderer Stelle zwischen der einführungsseitigen Öffnung des Katheterschlauchs und dem gegenüberliegenden Ende des Katheterschlauchs aus diesem hinausgeführt. Das Rücknahmeelement kann hierzu integral mit dem Katheterspitzenelement ausgebildet oder anderweitig mit diesem verbunden sein. Somit erstreckt sich das Rücknahmeelement vom Katheterspitzenelement ausgehend zumindest abschnittsweise durch den Katheterschlauch. Die Länge des Rücknahmeelements ist dabei mindestens so lang wie die Strecke zwischen dem Katheterspitzenelement und der Ausführungsöffnung für das dem Katheterspitzenelement abgewandte Ende des Rücknahmeelements unter weiterer Hinzunahme eines zum Greifen des Endes erforderlichen Streckenabschnitts. Sofern das Rücknahmeelement beispielsweise nicht manuell bewegt wird, sondern über einen entsprechenden Aktuator, kann die Länge des Rücknahmeelements auch nur zumindest bis zu diesem Aktuator reichen. Der Aktuator kann Teil des Katheterschlauchs sein oder auch separat von diesem ausgebildet sein.

Für eine Relativbewegung des gesamten Katheterspitzenelements greift das Rücknahmeelement bevorzugt an einem dem Katheterschlauch zugewandten Endabschnitt des Katheterspitzenelements an. Sofern nur ein Abschnitt des Katheterspitzenelements zur Überführung von der ersten Konfiguration in die zweite Konfiguration relativ bewegt wird, greift das Rücknahmeelement insbesondere an diesem Abschnitt an. Soll beispielweise das Katheterspitzenelement lediglich in Richtung des Katheterschlauchs nach innen gestülpt werden, kann das Rücknahmeelement an einem dem Katheterschlauch abgewandten Endabschnitt des Katheterspitzenelements angebunden sein, um direkt gezielt an dem einzustülpenden Ende angreifen zu können.

Insbesondere ist das Rücknahmeelement als Draht, Schnur oder Schlauch ausgebildet.

Über eine Draht, eine Schnur oder auch einen Schlauch kann das Rücknahmeelement ausreichend flexibel im Sinne einer Verformbarkeit gestaltet werden, um eine Einführung des Katheters bei schon im Katheterschlauch angeordnetem Rücknahmeelement nicht durch das Rücknahmeelement zu behindern.

Die Verwendung eines Drahts als Rücknahmeelement kann zudem trotz noch ausreichender Flexibilität auch eine notwendige Steifigkeit des Katheterschlauchs und/oder des Katheterspitzenelements unterstützen. Die Verwendung einer Schnur als Rücknahmeelement kann wiederum zur leichteren Handhabung der Relativbewegung Vorteile bieten. Über die Verwendung eines Schlauchs als Rücknahmeelement kann wiederum das Katheterspitzenelement bei entsprechender Ausgestaltung, wie vorstehend beschrieben, unabhängig vom Katheterschlauch mit einem positiven und/oder negativen Druck beaufschlagt werden. Grundsätzlich kann das Rücknahmeelement auch aus einer Kombination der vorgenannten Ausgestaltungen gebildet werden.

In einer Ausgestaltung ist die einführungsseitige Öffnung des Katheterschlauchs in der ersten Konfiguration durch das Katheterspitzenelement fluiddicht abdichtbar.

Das Katheterspitzenelement kann somit nicht nur der Einführung in ein Hohlorgan dienen, sondern kann die einführungsseitige Öffnung in der ersten Konfiguration auch fluiddicht abdichten, um beispielsweise einen frühzeitigen Fluidaustausch über den Katheterschlauch zu verhindern. Sofern das Katheterspitzenelement über den Katheterschlauch mit einem positiven oder negativen Druck beaufschlagbar ist, kann über die fluiddichte Abdichtung auch ein anderweitiger Druckverlust über die einführungsseitige Öffnung vermieden werden. Im Hinblick auf eine Druckbeaufschlagung kann das Katheterspitzenelement währenddessen insbesondere durch Rücknahmeelement in einer gewünschten Position gehalten werden.

Gemäß einer Weiterbildung wird die fluiddichte Abdichtung durch einen in Bezug auf die Erstreckungsrichtung des Katheterschlauchs radial zumindest abschnittsweise umlaufenden Vorsprung des Katheterspitzenelements gebildet, der insbesondere zumindest in radialer Richtung komprimierbar bzw. verkleinerbar ist.

Dieser radial zumindest abschnittsweise umlaufende Vorsprung kann die einführungsseitige Öffnung des Katheterschlauchs nicht nur fluiddicht abdichten, sondern auch als Anschlag für das Katheterspitzenelement in der ersten Konfiguration genutzt werden. In der ersten Konfiguration sitzt der Vorsprung demnach auf der einführungsseitigen Öffnung des Katheterschlauchs auf. Durch eine Komprimierbarkeit bzw. Verkleinerung des Vorsprungs in radialer Richtung kann der Anschlag bei der Überführung von der ersten Konfiguration in die zweite Konfiguration aufgehoben werden. Beispielweise wird der Vorsprung radial komprimiert bzw. verkleinert, wenn dieser zumindest abschnittsweise aus einem verformbaren Material gebildet wird, das aufgrund einer über ein vorstehend beschriebenes Rücknahmeelement aufgebrachten Zugkraft bei Relativbewegung des Katheterspitzenelements in den Katheterschlauch hinein bzw. durch die einführungsseitige Öffnung des Katheterschlauchs hindurch radial komprimiert wird. Alternativ oder ergänzend kann die Komprimierung auch durch die Beaufschlagung mit einem negativen Innendruck erfolgen. Zur Ausbildung der fluiddichten Abdichtung ist der radiale Vorsprung insbesondere vollständig radial umlaufend, um die Abdichtung im Wesentlichen über die Auflagefläche des Vorsprungs auf dem die einführungsseitige Öffnung umgebenen stirnflächenseitigen Rand auszubilden, wodurch Toleranzen einfacher auszugleichen sind.

In einer Ausgestaltung weist der Katheter einen Katheteranker auf, der in der Erstreckungsrichtung des Katheterschlauchs angrenzend an oder in einem vorbestimmten Abstand zur einführungsseitigen Öffnung angeordnet ist.

Der Begriff Katheteranker steht synonym für Katheterkomponenten, die der Verankerung des Katheters in einem Hohlorgan dienen. Ein Beispiel eines Katheterankers ist beispielsweise ein Katheterballon. Hierbei handelt es sich exemplarisch um einen in der Nähe der einführungsseitigen Öffnung angeordneten ringförmigen Abschnitt des Katheterschlauchs, der über eine Beaufschlagung mit einem positiven Innendruck ballonartig aufblasbar ist. Durch diese abschnittsweise Vergrößerung des Katherschlauchdurchmessers kann der Katheter in einem Hohlorgan gehalten werden.

Der Katheteranker kann somit unabhängig vom Katheterspitzenelement ausgebildet werden, so dass eine durch zumindest abschnittsweise Rücknahme des Katheterspitzenelements bedingte Veränderung keinen Einfluss auf die Halterung des Katheters in einem jeweiligen Hohlorgan hat. Zudem kann der Katheteranker gerade bei einer durch Zugkräfte bedingten zumindest abschnittsweisen Rücknahme des Katheterspitzenelements ein gleichzeitiges unbeabsichtigtes Herausziehen des Katheters bzw. des einführungsseitigen Endes des Katheterschlauchs aus einem Hohlorgan verhindern.

Gemäß einer Weiterbildung weist das Katheterspitzenelement eine Katheterspitzenelementöffnung auf, über die eine fluidische Verbindung zwischen einem die Katheterspitzenelementöffnung umgebenden Bereich, insbesondere einem Hohlorgan, und dem Katheterschlauch ausbildbar ist.

Die Katheterspitzenelementöffnung ermöglicht somit beispielsweise auch bei fluiddichter Abdichtung der einführungsseitigen Öffnung des Katheterschlauchs in der ersten Konfiguration eine fluidische Verbindung des die Katheterspitzenelementöffnung umgebenden Bereichs, also einer äußeren Umgebung, mit dem Katheterschlauch. Über die fluidische Verbindung kann somit auch in der ersten Konfiguration ein Fluidaustausch, beispielsweise zur Zuführung eines Gleitmittels während des Einführens des Katheters, zum Spülen des Hohlorgans oder der direkten Fluidableitung aus dem Hohlorgan nach der Einführung des Katheters, gewährleistet werden. Sofern das Katheterspitzenelement in der zweiten Konfiguration ohne Katheterspitzenelementöffnung eine fluidische Verbindung über die einführungsseitige Öffnung weiterhin blockieren würde oder könnte, kann dies zudem über die Katheterspitzenelementöffnung vermieden werden. Für die weitere Möglichkeit einer Druckbeaufschlagung des Katheterspitzenelements, wie vorstehend beschrieben, ist das Katheterspitzenelement in einer solchen Konstellation derart konfiguriert, dass der zur Druckbeaufschlagung vorgesehene Abschnitt des Katheterspitzenelements fluiddicht von der Katheterspitzenelementöffnung getrennt ist.

Alternativ oder ergänzend weist der Katheterschlauch neben der einführungsseitigen Öffnung zumindest eine zweite Öffnung auf, über die eine fluidische Verbindung zwischen einem die zweite Öffnung umgebenden Bereich, insbesondere einem Hohlorgan, und dem Katheterschlauch ausbildbar ist.

Über die zweite Öffnung kann beispielsweise unabhängig von der einführungsseitigen Öffnung bzw. deren Abdichtung oder Freigabe durch das Katheterspitzenelement eine fluidische Verbindung zu einem den Katheterschlauch umgebenden Bereich, wie zur Harnröhre während des Einführens oder zur Harnblase nach der Einführung des Katheters, geschaffen werden. Somit kann unter anderem in der ersten Konfiguration, bei der das Katheterspitzenelement die einführungsseitige Öffnung fluiddicht abdichtet, über die zweite Öffnung ein Gleitmittel nach außen geleitet werden, um die Einführung des Katheterschlauchs zu erleichtern. Auch kann nach der Einführung des Katheterschlauchs in ein Hohlorgan die erste Konfiguration in der vorstehenden Ausgestaltung zunächst beibehalten werden, um das Hohlorgan vorab über die zweite Öffnung zu spülen oder einen anderweitigen, von der einführungsseitigen Öffnung unabhängigen Fluidaustausch vorzusehen. Die einführungsseitige Öffnung kann bei Vorsehung einer zweiten Öffnung auch in der zweiten Konfiguration weiterhin durch das Katheterspitzenelement fluiddicht abgedichtet bleiben und muss somit aufgrund der vorhandenen Alternative nicht zwangsläufig zum Fluidaustausch geöffnet werden. Beispielsweise kann die einführungsseitige Öffnung des Katheterschlauchs lediglich zur Druckbeaufschlagung des Katheterspitzenelements mit einem positiven und/oder negativen Innendruck beaufschlagt werden. Sofern der einführungsseitigen Öffnung und der zweiten Öffnung gleiche Funktionen zuordenbar sind, diese aber nicht gleichzeitig ausgeführt werden sollen, kann der Katheter auch derart konfiguriert sein, dass in der ersten Konfiguration die zweite Öffnung geöffnet und die einführungsseitige Öffnung geschlossen ist, und durch die Überführung des Katheterspitzenelements von der ersten in die zweite Konfiguration die zweite Öffnung schließbar und die einführungsseitige Öffnung öffenbar ist. Zum Beispiel kann das Katheterspitzenelement die einführungsseitige Öffnung in der ersten Konfiguration fluiddicht abdichten und in der zweiten Konfiguration freigeben, während durch die Relativbewegung des Katheterspitzenelements die zweite Öffnung in der zweiten Konfiguration über das Katheterspitzenelement geschlossen wird.

In einer Ausgestaltung ist das Katheterspitzenelement zumindest teilweise aus einem fluidlöslichen Material gebildet und/oder ist hiermit zumindest teilweise beschichtet und/oder mit einem Medikament beschichtet ist.

Über das fluidlösliche Material kann der durch das in der ersten Konfiguration zumindest abschnittsweise aus oder von der einführungsseitigen Öffnung in eine dem Katheterschlauch abgewandten Richtung vorstehende Katheterspitzenelement gebildete Überstand zumindest in den das fluidlösliche Material ausweisenden Bereichen in die zweite Konfiguration überführt werden. Der Begriff der Fluidlöslichkeit des Materials kann eine Fluidlöslichkeit im Hinblick auf das im Hohlorgan anzutreffende Fluid, wie eine Harnflüssigkeit, und/oder eine Fluidlöslichkeit in Bezug auf ein speziell hierfür zugeführtes Fluid, wie beispielsweise über einen Spülvorgang, umfassen. Durch die zumindest teilweise Materialauflösung kann das Katheterspitzenelement ganz oder teilweise aufgelöst werden. Die Auflösung des Materials kann dabei von innen und/oder außen erfolgen. Infolgedessen können zumindest die Außenkontur des Katheterspitzenelements und somit damit verbundene Störkonturen verkleinert oder aufgelöst werden. Alternativ oder ergänzend ist es auch möglich, das Katheterspitzenelement zumindest soweit zu verkleinern oder die Steifigkeit durch die mit den Auflösungsvorgängen einhergehende Materialstärkenverringerung und/oder Materialschwächung zumindest soweit zu verringern, dass das Katheterspitzenelement durch eine Relativbewegung in die zweite Konfiguration überführbar ist. Die vorstehende chemische Auflösung kann somit auch mit den mechanischen Mitteln zur Überführung des Katheterspitzenelements von der ersten in die zweite Konfiguration kombiniert werden.

Die Verwendung einer fluidlöslichen Beschichtung kann insbesondere dazu dienen, verschiedene Materialeigenschaften für das Katheterspitzenelement in vorteilhafter Weise zu nutzen. Zum Beispiel kann eine vergleichsweise formstabiles Material als äußere Beschichtung mit relativ langsamen Auflösungsverhalten in dünner Schicht aufgetragen werden, wobei der darunterliegende Innenbereich des Katheterspitzenelements aus einem sich im Vergleich dazu schnell auflösenden Material mit geringerer Formbeständigkeit, aber mit Stützwirkung für die Beschichtung, gebildet wird. Die äußere Beschichtung gewährleistet dann eine geeignete Steifigkeit zur Einführung, wobei die äußere Beschichtung sich aufgrund des vergleichsweise dünnen Auftrags immer noch ausreichend schnell auflöst. Der darunterliegende Innenbereich muss an sich bei der Überführung in die zweite Konfiguration keine geeignete Steifigkeit mehr aufweisen und löst sich zudem nach Auflösung der äußeren Beschichtung schnell auf. Gemäß einem anderen exemplarischen Beispiel ist eine verformbare Außenhülle des Katheterspitzenelements von innen mit einer fluidlöslichen Beschichtung zur Bereitstellung einer geeigneten Steifigkeit versehen. Das Katheterspitzenelement kann in diesem Fall von innen mit einem auf die fluidlösliche Beschichtung zur Auflösung abgestimmten Fluid gespült werden, so dass sich die Beschichtung auflöst. Die verbleibende Außenhülle kann dann zum Beispiel derart verformt werden, dass sie durch eine Relativbewegung, wie vorstehend beschrieben, von der ersten Konfiguration in die zweite Konfiguration überführt werden kann. Sofern das Katheterspitzenelement die einführungsseitige Öffnung des Katheterschlauchs fluiddicht abdichtet und auch das Hohlorgan anderweitig gegen das Eindringen des in dieser Ausgestaltung genutzten Fluids geschützt ist, können auch Fluide bzw. Material-Fluid-Kombination verwendet werden, die lediglich auf ihr Auflösungsverhalten abgestimmt sein können. Sicherheitsaspekte kommen in diesem Zusammenhang primär in Bezug auf mögliche Undichtigkeiten in Betracht, nicht aber in erster Linie in Bezug auf eine Verträglichkeit in Bezug auf das Hohlorgan an sich.

Alternativ oder ergänzend zum Einsatz eines fluidlöslichen Materials für das Katheterspitzenelement oder als Beschichtung für das Katheterspitzenelement kann auch ein Material vorgesehen werden, bei dem der Kontakt mit einem Fluid, wie beispielweise der Harnflüssigkeit, eine Materialschwächung oder Materialaufweichung bewirkt, durch die eine mechanische Materialzersetzung durch äußere Krafteinwirkung, wie durch die Rückziehbewegung, oder eine Verringerung der Steifigkeit zu einem vergleichbaren Ergebnis führt.

Weiterhin alternativ oder ergänzend kann das Katheterspitzenelement mit einem Medikament beschichtet sein. Beispielsweise können somit über das Katheterspitzenelement entzündungshemmende Substanzen abgegeben werden.

Insbesondere ist der Katheter ein Blasenkatheter, beispielsweise ein transurethraler Blasenkatheter.

Gerade Blasenkatheter werden häufig zum Verbleib über einen längeren Zeitraum eingesetzt. Die eingangs genannten Probleme durch Organschrumpfung bei gleichzeitiger Bewegungstendenz des Patienten treten hier in besonderen Maßen auf. Dies betrifft, ebenfalls wie eingangs beschrieben, insbesondere transurethrale Blasenkatheter. In diesem Zusammenhang kann aber auch ein suprapubischer Blasenkatheter durch Ergänzung mit einem entsprechenden Katheterspitzenelement zu einem transurethralen Blasenkatheter ausgebildet werden. Umgekehrt kann der transurethrale Blasenkatheter durch Entfernung des Katheterspitzenelements als suprapubischer Blasenkatheter eingesetzt werden. Somit sind nicht zwangsläufig zwei unterschiedliche Blasenkathetertypen zu bevorraten, sondern es könnte ein Blasenkatheter in Abhängigkeit der finalen Ausgestaltung für beide Anwendungen verwendet werden. Hierfür bietet sich insbesondere eine zweiteilige Ausführung des Katheterschlauchs und des Katheterspitzenelements an, wobei Letzteres dann lediglich bedarfsweise in den Katheterschlauch eingeführt wird.

Ein weiterer Aspekt der Offenbarung betrifft ein Katheterspitzenelement, das dazu konfiguriert ist, ein Katheterspitzenelement für einen vorstehend beschriebenen Katheter auszubilden.

In Anlehnung an den vorgehenden Abschnitt kann somit beispielsweise ein suprapubischer Blasenkatheter zur Verwendung als transurethraler Katheter nachgerüstet werden. Bei der zweiteiligen Ausgestaltung von Katheterspitzenelement und Katheterschlauch können sich zudem flexible Kombinationsmöglichkeiten ergeben, durch die das Anwendungsspektrum bei reduzierter Teilezahl erweitert werden kann.

Zudem betrifft ein Aspekt der Offenbarung auch ein Katheter-Set, umfassend zumindest ein Katheterspitzenelement und einen Katheterschlauch, der dazu konfiguriert ist, zusammen mit dem Katheterspitzenelement einen erfindungsgemäßen Katheter auszubilden.

Das Katheter-Set ermöglicht die bereits angeführten Vorteile der flexiblen Nutzung für unterschiedliche Anwendungstypen, beispielsweise als transurethraler oder suprapubischer Blasenkatheter, und/oder die Verwendung bedarfsgerechter unterschiedlicher Katheterspitzenelemente.

Im Folgenden wird die Erfindung unter Bezugnahme auf die beigefügten Figuren näher erläutert. Die Figuren zeigen im Einzelnen:
Figur 1 eine schematische Darstellung eines in eine Harnblase eingeführten transurethralen Blasenkatheters gemäß eines bekannten Stands der Technik;
Figur 2 eine ausschnittsweise schematische Querschnittsansicht einer ersten Ausführungsform eines erfindungsgemäßen Katheters parallel zur Erstreckungsrichtung eines Katheterschlauchs;
Figur 3 eine ausschnittsweise schematische Querschnittsansicht einer zweiten Ausführungsform eines erfindungsgemäßen Katheters parallel zur Erstreckungsrichtung eines Katheterschlauchs sowie eine ausschnittsweise Querschnittsansicht entlang der Linie A-A;
Figur 4 eine ausschnittsweise schematische Querschnittsansicht einer dritten Ausführungsform eines erfindungsgemäßen Katheters parallel zur Erstreckungsrichtung eines Katheterschlauchs sowie eine Querschnittsansicht entlang der Linie A-A; und
Figur 5 eine ausschnittsweise schematische Querschnittsansicht einer vierten, nicht erfindungsgemäßen

Ausführungsform eines Katheters, parallel zur Erstreckungsrichtung eines Katheterschlauchs.

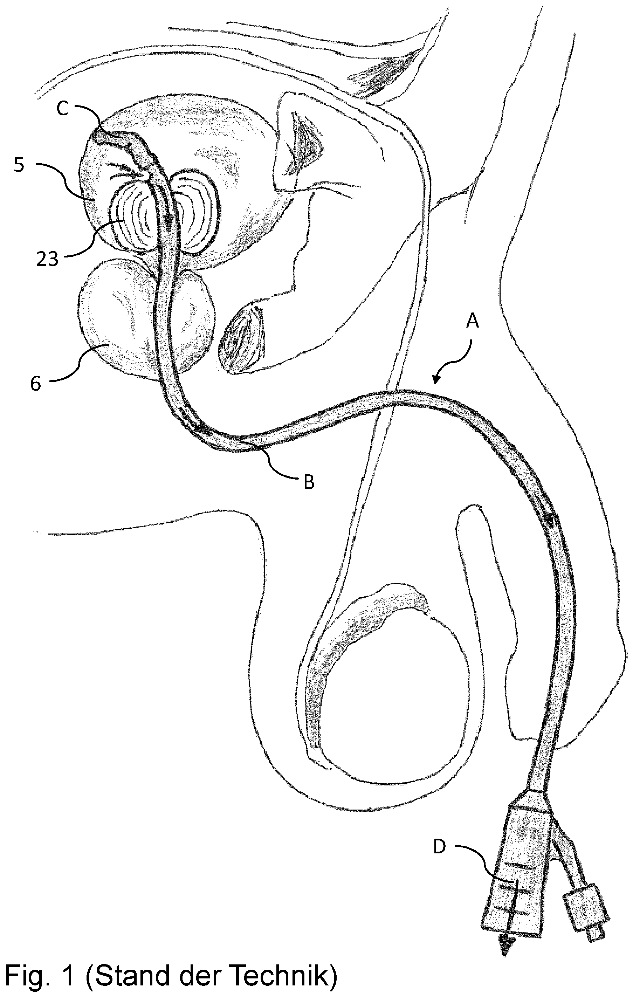

Zunächst wird exemplarisch anhand eines transurethralen Blasenkatheters der Stand der Technik als Orientierungshilfe beschrieben. Wie in **Figur 1** dargestellt, umfasst ein Blasenkatheter A einen Katheterschlauch B, eine Katheterspitze C sowie einen Katheteradapter D. Der Katheterschlauch B wird unter Zuhilfenahme der Katheterspitze C durch die Harnröhre eines (hier männlichen) Patienten eingeführt, bis eine Öffnung des Katheterschlauchs B zur Ableitung einer Harnflüssigkeit die Harnblase 5 erreicht. Die Katheterspitze C ist hier als sogenannte Tiemann-Spitze ausgebildet und weist eine geeignete Steifigkeit auf, um die Einführung des Katheterschlauchs in die Harnblase zu ermöglichen. Die geeignete Steifigkeit bezieht sich dabei sowohl auf die Einführung durch die Harnröhre an sich als auch die Vorbeiführung an die Harnröhre umgebenden Organen, wie hier der Prostata 6. Zudem umfasst der Katheterschlauch einen Katheterballon 23 als Katheteranker, um den in die Harnblase eingeführten Bereich des Katheterschlauchs B in der Harnblase zu halten. Durch Beaufschlagung des Katheterballons 23 mit einem positiven Innendruck wird dieser auf einen im Vergleich zur Harnröhre vergrößerten Durchmesser ballonartig aufgebläht, so dass der Katheterschlauch B nicht mehr ohne weiteres aus der Harnblase 5 herausgezogen werden kann. Die ordnungsgemäße Entfernung des Katheterschlauchs erfolgt dann zum jeweiligen Zeitpunkt über die Rücknahme der Druckbeaufschlagung des Katheterballons 23. Über die zwischen dem Katheterschlauch der Katheterspitze C und dem Katheterballon 23 angeordnete Öffnung kann bei eingeführtem Blasenkatheter A Harnflüssigkeit aus der Harnblase abgeleitet werden. Diese wird über den Katheteradapter D in ein daran befestigtes Fluidaufnahmebehältnis (nicht gezeigt) weitergeleitet. Der Katheteradapter kann weitere Anschlüsse aufweisen, um beispielsweise hierüber die Druckbeaufschlagung des Katheterballons 23 vorzunehmen und/oder eine Spülflüssigkeit in den Katheterschlauch bzw. folglich die Harnblase einzuleiten.

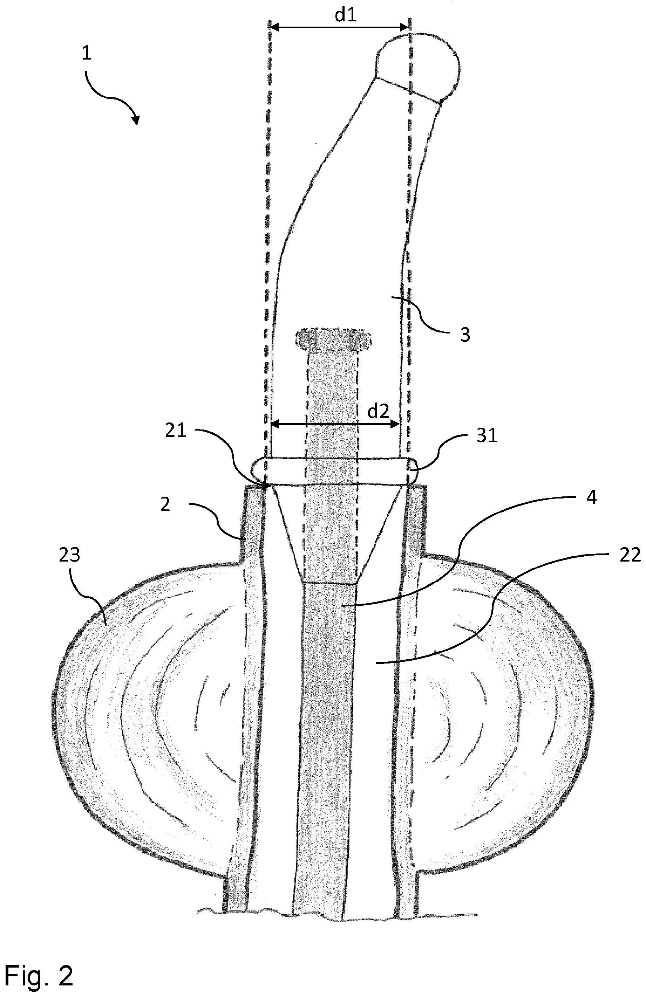

Wie in Figur 1 dargestellt, kann die Katheterspitze C des Blasenkatheters A gemäß dem Stand der Technik, insbesondere bei Schrumpfung der Harnblase durch kontinuierliche Ableitung der Harnflüssigkeit, mit der Innenseite der Harnblase 5 in Kontakt kommen. Durch Relativbewegungen zwischen der Innenseite der Harnblase 5 und der Katheterspitze C und die damit verbundene Reibung können Reizungen bis hinzu Verletzungen der Schleimhaut auftreten. Dies kann durch einen erfindungsgemäßen Katheter, wie er exemplarischen anhand der ersten, zweiten und dritten Ausführungsform unter Bezugnahme auf die Figuren 2 bis 4 folgend beschrieben wird, verhindert oder zumindest ein entsprechendes Risiko minimiert werden.

**Figur 2** zeigt hierzu eine ausschnittsweise schematische Querschnittsansicht einer ersten Ausführungsform eines erfindungsgemäßen Katheters 1 parallel zur Erstreckungsrichtung eines Katheterschlauchs 2. Der Katheter 1 weist einen Katheterschlauch 2 mit einer einführungsseitigen Öffnung 21 sowie einem Katheterballon 23, wie er auch zu Figur 1 beschrieben wird, auf. Die einführungsseitige Öffnung 21, also das in Erstreckungsrichtung des Katheterschlauchs 2 zur Einführung vorgesehene Ende des Katheterschlauchs 2, weist einen Innendurchmesser auf, der hier einem Innendurchmesser eines Rücknahmeabschnitts 22 entspricht. Der Rücknahmeabschnitt 22 korrespondiert in dieser Ausführungsform zum Innendurchmesser des gesamten Katheterschlauchs 2, kann aber auch nur durch einen Abschnitt des Katheterschlauchs 2 gebildet sein.

Zudem umfasst der Katheter 1 ein Katheterspitzenelement 3. Das Katheterspitzenelement 3 ist hier in einer ersten Konfiguration gezeigt, in der ein Abschnitt des Katheterspitzenelements 3 aus der einführungsseitigen Öffnung 21 in eine dem Katheterschlauch 2 abgewandten Richtung vorsteht. Der dadurch gebildete Überstand ist leicht abgewinkelt und weist eine geeignete Steifigkeit auf, um zusammen mit dem sich an den Überstand anschließenden Katheterschlauch 2 in ein Hohlorgan, wie die Harnblase 5, einführbar zu sein. Gleichzeitig ist zumindest der abgewinkelte Bereich ausreichend flexibel im Sinne einer Verformbarkeit, um bei einer Überführung von der ersten Konfiguration in eine zweite Konfiguration, wie dies folgend noch beschrieben wird, überführt werden zu können. Das Katheterspitzenelement 3 weist zudem einen in Bezug auf Erstreckungsrichtung des Katheterschlauchs 2 radial nach außen weisenden, umlaufenden Vorsprung 31 auf. Dieser ist in Bezug auf die Erstreckungsrichtung des Katheterschlauchs 2 zumindest radial komprimierbar, wobei der Außenmesser des radial umlaufenden Vorsprungs 31 in radialer Richtung in einem nichtkomprimierten Zustand größer als der Innendurchmesser, hier gleich dem Innendurchmesser d1 des Rücknahmeabschnitts 22, der einführungsseitigen Öffnung 21 ist. Der radial umlaufende Vorsprung 31 dient in der ersten Konfiguration als Anschlag für das Katheterspitzenelement 3, um beim Einführen in die Harnröhre ein unerwünschtes nach innen Bewegen des Katheterspitzenelements 3 zu verhindern. Ausgehend vom radial umlaufenden Vorsprung 31 erstreckt sich ein trichterförmiger Abschnitt des Katheterspitzenelements 3 in Erstreckungsrichtung des Katheterschlauchs 2 nach innen in diesen hinein. Durch die trichterförmige Ausgestaltung dieses Abschnitts kann das Katheterspitzenelement 3 bis zum Erreichen des Anschlags in einfacher Weise zentrierend über die einführungsseitige Öffnung 21 in den Katheterschlauch 2 eingeführt werden. Darüber hinaus weist das Katheterspitzenelement 3 ein Rücknahmeelement 4 auf, das in dem Katheterspitzenelement 3 befestigt ist und sich von dem trichterförmigen Abschnitt ausgehend durch den Katheterschlauch 2 erstreckt. Das Rücknahmeelement 4 kann ein Draht, ein Schlauch, eine Schnur oder auch ein andere flexibles, langgestrecktes Element sein, das in Erstreckungsrichtung des Katheterschlauchs 2 relativ zu diesem bewegbar ist.

Zur Überführung des Katheterspitzenelements 3 von der ersten Konfiguration in die zweite Konfiguration, in der der durch das Katheterspitzenelement 3 gebildete Überstand aufgehoben oder zumindest reduziert wird, wird nun über das Rücknahmeelement 4 eine Zugkraft auf das Katheterspitzenelement 3 in eine dem Katheterspitzenelement 3 entgegengesetzte Richtung aufgebracht. Durch die aufgebrachte Zugkraft wird das Katheterspitzenelement 3 weiter in den Katheterschlauch 2 hinein gedrängt, wodurch der radial umlaufende Vorsprung 31 auf einen Durchmesser komprimiert wird, der kleiner oder gleich dem Innendurchmesser der einführungsseitigen Öffnung bzw. dem Innendurchmesser d1 des Rücknahmeabschnitts 22 ist. Der sich von dem radialen umlaufenden Vorsprung 31 aus erstreckende Überstand des Katheterspitzenelements 3 weist zudem einen maximalen äußeren Durchmesser d2 auf, der ebenfalls kleiner oder gleich dem Innendurchmesser der einführungsseitigen Öffnung bzw. dem Innendurchmesser d1 des Rücknahmeabschnitts 22 ist. Somit kann das Katheterspitzenelement 3 relativ zum Katheterschlauch 2 in den Rücknahmeabschnitt 22 bewegt werden. Der Rücknahmeabschnitt 22 ist der Abschnitt, der zur Aufnahme des Katheterspitzenelements 3 in den Katheterschlauch vorgesehen ist. Demnach erstreckt sich der Rücknahmeabschnitt 22 von der einführungsseitigen Öffnung 21 in Erstreckungsrichtung des Katheterschlauchs 2 in einer der einführungsseitigen Öffnung 21 abgewandten Richtung durch den Katheterschlauch 2 zumindest über die für die Aufnahme des Katheterspitzenelements 3 vorgesehene Länge. In der vorliegenden Ausführungsform ist das Katheterspitzenelement 3 durch den Katheterschlauch 2 aus diesem über eine Ausführungsöffnung (nicht gezeigt) für das Katheterspitzenelement 3 herausziehbar, so dass die Länge des Rücknahmeabschnitts 22 der Länge des Katheterschlauchs 2 von der einführungsseitigen Öffnung 21 bis zur vorgenannten Ausführungsöffnung entspricht. Grundsätzlich ist hierzu der minimale Innendurchmesser d1 des Rücknahmeabschnitts 22 über dessen Länge während der Überführung des Katheterspitzenelements 3 von der ersten Konfiguration in die zweite Konfiguration nicht kleiner als der maximale Außendurchmesser d2 des in dem Rücknahmeabschnitt 22 aufzunehmenden Abschnitts des Katheterspitzenelements 3. In der gezeigten Ausführungsform ist der minimale Innendurchmesser des Rücknahmeabschnitts 22 auch nicht kleiner als der maximale Außendurchmesser des radial umlaufenden Vorsprungs 31 im komprimierten Zustand. Der minimale Innendurchmesser d1 des Rücknahmeabschnitts 22 bezieht sich dabei auf den minimalen Innendurchmesser während der Überführung des Katheterspitzenelements 3 von der ersten Konfiguration in die zweite Konfiguration. Demnach kann der minimale Innendurchmesser d1 ein Durchmesser sein, der schon im Ausgangszustand bezüglich der ersten Konfiguration vorliegt oder der sich während der Überführung bzw. Relativbewegung durch elastische Verformungsanteile ergibt.

Die elastische Verformung kann beispielsweise durch das Katheterspitzenelement 3 bedingt sein und/oder in einer weiteren Ausführungsform durch eine Beaufschlagung des Rücknahmeabschnitts 22 mit einem positiven Innendruck unterstützt werden. In diesem Fall ist der umlaufende Vorsprung 31 abdichtend ausgeführt bzw. erfüllt zusätzlich eine Abdichtfunktion.

Bei der in Figur 1 gezeigten Ausführungsform ist der Katheterschlauch 2 aus einem flexiblen Material gebildet und weist über seine gesamte Länge einen konstanten Innendurchmesser aus, so dass der minimale Innendurchmesser d1 des Rücknahmeabschnitts 22 durch den Innendurchmesser des Katheterschlauchs 2 gebildet wird und somit ebenfalls konstant ist.

Über das vorstehend beschriebene Durchmesserverhältnis des minimalen Innendurchmessers d1 des Rücknahmeabschnitts 22 zum maximalen Außendurchmesser d2 des Katheterspitzenelements 3 kann das Katheterspitzenelement 3 gemäß der durch das Rücknahmeelement 4 aufgebrachten Zugkraft nun durch die einseitige Öffnung 21 in den Katheterschlauch 2 bzw. den Rücknahmeabschnitt 22 hineinbewegt werden. Der Überstand des Katheterspitzenelements 3 aus der einführungsseitigen Öffnung 21 reduziert sich dabei entsprechend der durch das Rücknahmeelement 4 durchgeführten Rückziehbewegung. Zu dem dem Rücknahmeelement 4 gegenüberliegenden abgewinkelten Ende des Katheterspitzenelements 3 ist ergänzend anzumerken, dass dieses zur Einführung in den Rücknahmeabschnitt 22 eine geringere Steifigkeit als der Rücknahmeabschnitt 22 aufweist, um sich zur Aufnahme in den Rücknahmeabschnitt 22 geeignet zu verformen, also eine ausreichende Rücknahme der Winkels vornehmen zu können. Alternativ oder ergänzend kann auch der Rücknahmeabschnitt 22 ausreichend flexibel sein, um den abgewinkelten Abschnitt nachzuformen. In einer weiteren Alternative kann die Länge des abgewinkelten Abschnitts aber auch derart dimensioniert sein, dass diese als verbleibender Überstand nur noch ein geringes Risiko eines Kontakts mit der Innenwand eines Hohlorgans birgt und somit in der zweiten Konfiguration nicht durch den Rücknahmeabschnitt 22 aufgenommen werden muss.

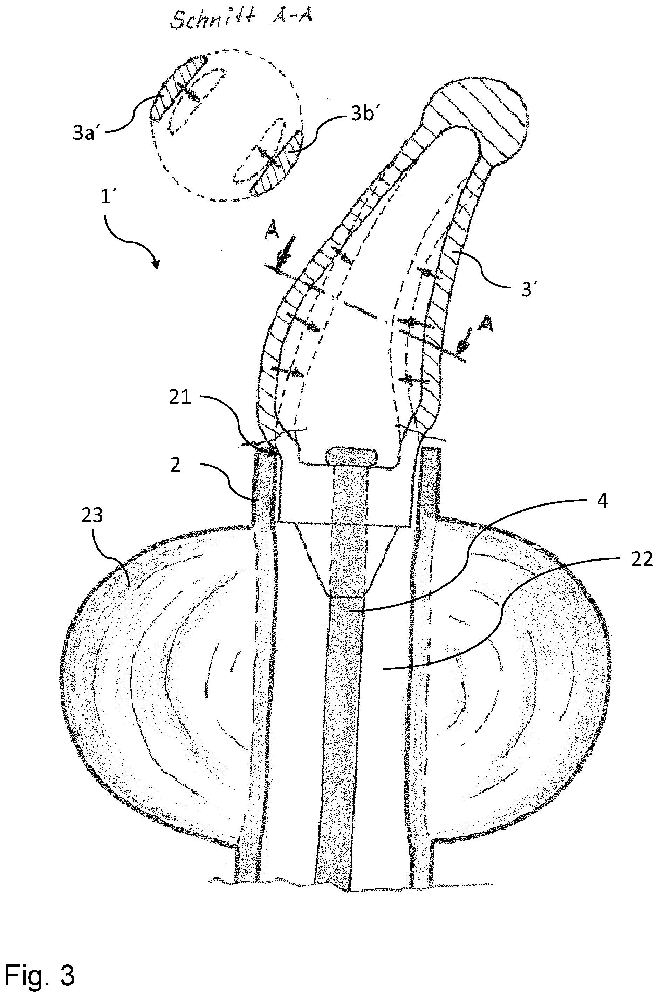

**Figur 3** zeigt eine ausschnittsweise schematische Querschnittsansicht einer zweiten Ausführungsform eines erfindungsgemäßen Katheters 1' parallel zur Erstreckungsrichtung eines Katheterschlauchs 2 sowie eine ausschnittsweise Querschnittsansicht entlang der Linie A-A. Der Katheter 1' der zweiten Ausführungsform unterscheidet sich von dem Katheter 1 der ersten Ausführungsform durch die Ausgestaltung des Katheterspitzenelements 3'. Für ansonsten korrespondierende Merkmale des Katheters 1' werden die Bezugszeichen gemäß Figur 1 zur ersten Ausführungsform verwendet.

Das Katheterspitzenelement 3' des Katheters 1' ist hier als verformbarer Hohlkörper ausgebildet. Der Hohlkörper weist in der gezeigten Ausführungsform eine verformbare Außenhülle sowie zwei Stege 3a', 3b' (Schnitt A-A) auf, die jeweils das dem Katheterschlauch 2 abgewandte Ende des Katheterspitzenelements 3' mit dem dem Katheterschlauch 2 zugewandten Ende des Katheterspitzenelements 3' verbinden. Die Stege 3a', 3b' sind federelastisch und weisen eine zur Einführung des Katheters 1' geeignete Steifigkeit auf.

Alternativ kann das Katheterspitzenelement 3' auch als Vollkörper mit entsprechenden Materialeigenschaften ausgebildet sein. Die Verformbarkeit des Katheterspitzenelements 3' bedingt sich hier durch ein federelastisches Material, aus dem des Katheterspitzenelement 3' gebildet wird. Durch das federelastische Material ist der maximale Außendurchmesser d2 (wie in Fig. 2) des Katheterspitzenelements 3' anpassbar. Diesbezüglich wäre es grundsätzlich auch möglich, lediglich den Bereich des Katheterspitzenelements 3' federelastisch auszubilden, für den eine Anpassbarkeit des maximalen Außendurchmessers d2 vorzusehen ist. Durch die federelastische Ausgestaltung des Katheterspitzenelements 3' kann eine ausreichende Steifigkeit für die Einführung des Katheters 1' in ein Hohlorgan vorgesehen werden. Selbst wenn während der Einführung von außen Druckkräfte auf das Katheterspitzenelement 3' wirken, kann diesen eine vorbestimmte Federkraft entgegengesetzt werden.

Die durchgezogenen Linien des Katheterspitzenelements 3' zeigen das Katheterspitzenelement 3' in der ersten Konfiguration. Der maximale Außendurchmesser des Katheterspitzenelements 3' ist dabei größer als der Innendurchmesser der einführungsseitigen Öffnung 21 und der minimale Innendurchmesser d2 des Rücknahmeabschnitts 22, wobei auch hier die jeweiligen Innendurchmesser gleich sind. Das Katheterspitzenelement 3' sitzt somit auf der einführungsseitigen Öffnung 21 auf. Auch hier ist das dem Katheterschlauch 2 zugewandte Ende des Katheterspitzenelements 3' mit einem Rücknahmeelement 4 verbunden.

Zur Überführung des Katheterspitzenelements 3' in die zweite Konfiguration wird nun vergleichbar zur ersten Ausführungsform das Rücknahmeelement 4 in eine der einführungsseitigen Öffnung 21 abgewandte Richtung bewegt. Durch die dadurch aufgebrachten Zugkräfte erfolgt eine hierzu korrespondierende Bewegung des Katheterspitzenelements 3' in Richtung des Rücknahmeabschnitts 22. In Zusammenwirken der federelastischen Stege 3a', 3b' und der verformbaren Außenhülle des Katheterspitzenelements 3' mit der einführungsseitigen Öffnung 21 wird der maximale Außendurchmesser des Katheterspitzenelements 3' auf einen Durchmesser komprimiert, der kleiner oder gleich dem minimalen Innendurchmesser des Rücknahmeabschnitts 22 ist. Die Komprimierung bzw. Eindrückung der Stege 3a', 3b' ist in Figur 3 über die gestrichelten Linien dargestellt. Die eingezeichneten Pfeile verdeutlichen die Verformung des Katheterspitzenelements 3' ausgehend von der ersten Konfiguration zur Überführung in die zweite Konfiguration. Die gezeigte Verformung muss sich dabei nicht direkt mit Beginn der Bewegung des Katheterspitzenelements 3' in den Rücknahmeabschnitt 22 ergeben, sondern kann sich auch erst über die Bewegungstrecke final ergeben. Zur Vereinfachung ist die Verformung jedoch in Figur 3 ungeachtet des bewegungsabhängigen Verlaufs direkt mit eingetragen. Grundsätzlich kann die Verformung des Katheterspitzenelements 3' zur Überführung in die zweite Konfiguration sowohl lediglich elastisch als auch plastisch sein. Durch plastische Verformungsanteile kann eine Wiederverwertung des Katheterspitzenelements 3' verhindert werden.

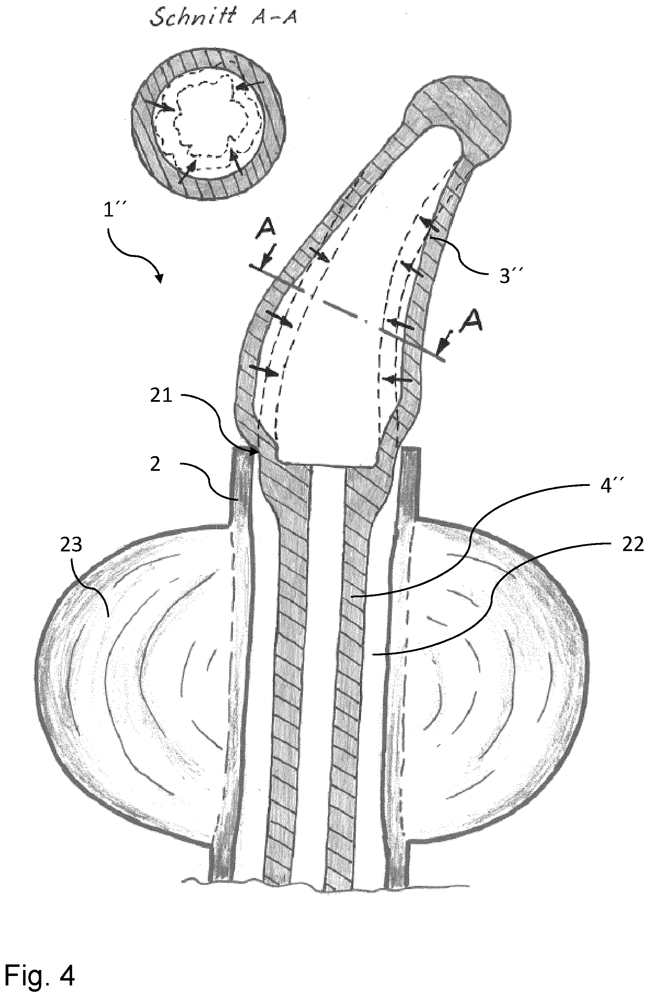

**Figur 4** zeigt eine ausschnittsweise schematische Querschnittsansicht einer dritten Ausführungsform eines erfindungsgemäßen Katheters 1" parallel zur Erstreckungsrichtung eines Katheterschlauchs 2 sowie eine Querschnittsansicht entlang der Linie A-A. Der Katheter 1" der dritten Ausführungsform unterscheidet sich von dem Katheter 1 der ersten Ausführungsform und dem Katheter 1' der zweiten Ausführungsform durch die Ausgestaltung des Katheterspitzenelements 3" in Zusammenwirken mit dem Rücknahmeelement 4". Für ansonsten korrespondierende Merkmale des Katheters 1" werden die Bezugszeichen gemäß Figur 1 zur ersten Ausführungsform verwendet.

Das Katheterspitzenelement 3" ist hier als Hohlkörper ausgebildet. Das Innere des Hohlkörpers des Katheterspitzenelements 3" steht dabei in fluidischer Verbindung mit einem als Schlauch ausgebildeten Rücknahmeelement 4". Hier ist das Rücknahmeelement 4" integral mit dem Katheterspitzenelement ausgebildet, kann aber auch anderweitig mit dem Katheterspitzenelement 3" verbunden werden. Die integrale Ausbildung minimiert das Risiko möglicher Leckagen durch Verbindungstoleranzen. Über das Rücknahmeelement 4" kann der Innenraum des Hohlkörpers des Katheterspitzenelements 3" mit einem positiven und/oder negativen Innendruck beaufschlagt werden. Das Rücknahmeelement 4" dient somit einer Doppelfunktion, d.h. der Ausübung einer Rücknahmebewegung und der Druckbeaufschlagung. Grundsätzlich können diese Funktionen aber auch getrennt werden, beispielsweise durch ein Rücknahmeelement gemäß den Figuren 1 und 2 und einer Druckbeaufschlagung über den Katheterschlauch 2, bei entsprechender fluidischer Verbindung zum Innenraum des Hohlkörpers des Katheterspitzenelements 3".

In der ersten Konfiguration weist der Überstand des Katheterspitzenelements 3" aus der einführungsseitigen Öffnung 21 eine geeignete Steifigkeit zur Einführung in ein Hohlorgan auf. Die geeignete Steifigkeit wird über eine entsprechende Druckbeaufschlagung mit einem positiven Innendruck des Innenraums des Hohlkörpers des Katheterspitzenelements 3" vorgesehen. In einer alternativen Ausgestaltung kann die geeignete Steifigkeit auch über das Material des Katheterspitzenelements selbst oder das Material des Katheterspitzenelements in Verbindung mit einer unterstützenden Druckbeaufschlagung mit einem positiven Innendruck vorgesehen werden. Im Übrigen ergibt sich bezüglich des Sitzes des Katheterspitzenelements 3" in der einführungsseitigen Öffnung 21 eine zur zweiten Ausführungsform in der ersten Konfiguration vergleichbare Konstellation.

Zur Überführung des Katheterspitzenelements 3" in die zweite Konfiguration wird der Innendruck im Innenraum des Hohlkörpers des Katheterspitzenelements 3" reduziert. Hierzu wird beispielsweise ein zuvor eingebrachter positiver Innendruck zumindest teilweise aufgehoben, um eine ausreichende Verformung des Katheterspitzenelements 3" für eine Aufnahme in den Rücknahmeabschnitt 22 bei entsprechender Ziehbewegung des Rücknahmeelements 4" zu ermöglichen. Die Überführung des Katheterspitzenelements 3" erfolgt dann vergleichbar zur zweiten Ausführungsform.

Alternativ oder ergänzend kann der Innenraum des Hohlkörpers des Katheterspitzenelements 3" aber auch gezielt mit einem negativen Innendruck beaufschlagt werden, so dass die für die Aufnahme in den Rücknahmeabschnitt 22 erforderliche Verformung des Katheterspitzenelements 3" durch den negativen Innendruck erfolgt bzw. hierdurch unterstützt wird. Die Querschnittsansicht entlang der Linie A-A zeigt anhand der gestrichelten Linien eine solche durch negative Druckbeaufschlagung hervorgerufene Komprimierung des Katheterspitzenelements 3" korrespondierend zu den auch in der Querschnittsansicht entlang der Erstreckungsrichtung gestrichelt eingezeichneten Durchmesseranpassungen. Durch die Komprimierung des Katheterspitzenelements 3" gemäß einer negativen Druckbeaufschlagung kann der maximale Außendurchmesser des Katheterspitzenelements 3", der in den Rücknahmeabschnitt 22 aufzunehmen ist, in Abhängigkeit von der Höhe des negativen Drucks an verschiedene minimale Innendurchmesser des Rücknahmeabschnitts 22 angepasst werden. Die Verformung des Katheterspitzenelements 3" kann sowohl als auch nach Initiierung der Rückziehbewegung über das Rücknahmeelement 4" erfolgen.

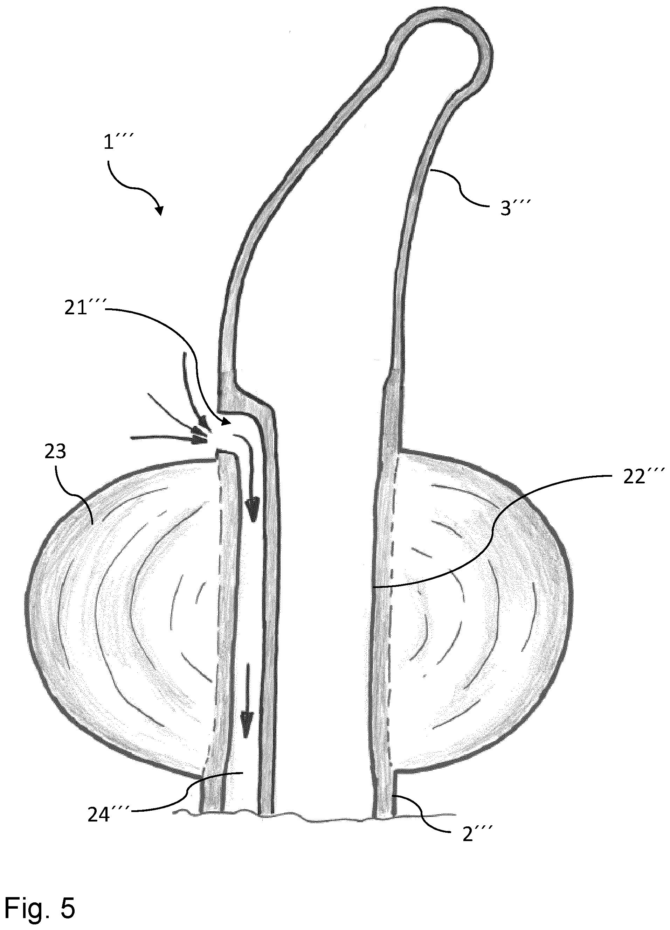

Figur 5 zeigt eine ausschnittsweise schematische Querschnittsansicht einer vierten, nicht erfindungsgemäßen Ausführungsform eines Katheters 1‴, parallel zur Erstreckungsrichtung eines Katheterschlauchs 2"'. Der Katheter 1‴ weist ein Katheterspitzenelement 3‴ auf, das gemäß der vierten Ausführungsform integral mit einem Katheterschlauch 2‴ ausgebildet ist. Demnach erstreckt sich das Katheterspitzenelement nicht aus einer einführungsseitigen Öffnung 21 "', sondern von der einführungsseitigen Öffnung 21‴ in eine dem Katheterschlauch 2‴ abgewandte Richtung. Die einführungsseitige Öffnung 21‴ ist hier radial zur Erstreckungsrichtung des Katheterschlauchs 2‴ zwischen dem Katheterspitzenelement 3‴ und dem Katheterballon 23 angeordnet.

Der Katheterschlauch 2‴ weist einen Fluidkanal 24‴ auf, über den in Verbindung mit der einführungsseitigen Öffnung 21‴ ein Fluidaustausch, wie beispielsweise eine Ausleitung von Harnflüssigkeit, erfolgen kann, wie dies durch die in Figur 5 eingezeichneten Pfeile angedeutet wird. Hiervon getrennt weist der Katheterschlauch 2‴ einen einen Rücknahmeabschnitt 22‴ umfassenden weiteren Kanal auf, über den das Katheterspitzenelement 3‴ mit einem Druck beaufschlagbar ist. Hierzu ist das Katheterspitzenelement 3‴ als verformbarer Hohlkörper ausgebildet. In der ersten Konfiguration wird das Innere des Hohlkörpers des Katheterspitzenelements 3‴ zur Einführung in ein Hohlorgan mit einem positiven Druck beaufschlagt, über den eine für die Einführung ausreichende Steifigkeit des Katheterspitzenelements 3‴ erreicht wird. Zur Überführung von der ersten in die zweite Konfiguration wird dann das Innere des Hohlkörpers des Katheterspitzenelements 3‴ mit einem negativen Druck beaufschlagt, so dass sich das Katheterspitzenelement 3‴ in den Rücknahmeabschnitt 22‴ nach innen einstülpt. In einer alternativen Ausführungsform kann zur Überführung in die zweite Konfiguration lediglich der positive Innendruck reduziert oder aufgehoben werden, wobei das Katheterspitzenelement 3‴ derart konfiguriert ist, dass es bei reduziertem oder aufgehobenen Innendruck in sich zusammenfällt. Das Katheterspitzenelement 3‴ bildet in diesem Fall zumindest keine wesentliche Störkontur mehr auf und/oder ist in seiner Steifigkeit derart reduziert, dass keine wesentliche Reibung mit dem Inneren des Hohlorgans auftritt.

Die Erfindung ist nicht auf die beschriebenen Ausführungsformen beschränkt. Grundsätzlich sind dabei alle beschriebenen Merkmale auch ungeachtet spezifischer Ausführungsformen untereinander kombinierbar, sofern sich eine entsprechende Kombination nicht vernünftigerweise ausschließt. Insbesondere kann eine Formänderung eine Katheterspitzenelements im Hinblick auf einen verbleibenden Überstand und/oder eine Durchmesserreduzierung auch durch fluidlösliche Materialien, die durch Körperflüssigkeiten oder anderweitig eingebrachte Substanzen chemisch zersetzt werden, erfolgen oder unterstützt werden.

### Bezugszeichenliste

- 1, 1', 1", 1‴: Katheter
- 2, 2‴: Katheterschlauch
- 3, 3', 3", 3‴: Katheterspitzenelement
- 3a', 3b': Steg (Katheterspitzenelement)
- 4, 4": Rücknahmeelement
- 21, 21‴: einführungsseitige Öffnung (Katheterschlauch)
- 22, 22‴: Rücknahmeabschnitt (Katheterschlauch)
- 23: Katheterballon (Katheteranker)
- 24‴: Fluidkanal
- 31: radial umlaufender Vorsprung (Katheterspitze)
- 5: Harnblase
- 6: Prostata
- A: Blasenkatheter (Stand der Technik)
- B: Katheterschlauch (Stand der Technik)
- C: Katheterspitze (Stand der Technik)
- D: Katheteradapter (Stand der Technik)
- d1: minimaler Innendurchmesser (Rücknahmeabschnitt)
- d2: maximaler Durchmesser (Katheterspitze)

## Patentansprüche

1. Katheter (1, 1', 1", 1‴), umfassend:
einen flexiblen Katheterschlauch (2, 2‴) mit einer einführungsseitigen Öffnung (21, 21‴) an einem einführungsseitigen Ende des Katheterschlauchs (2, 2‴), und ein Katheterspitzenelement (3, 3', 3", 3‴), das derart konfiguriert ist, dass das Katheterspitzenelement (3, 3', 3", 3‴) von einer ersten Konfiguration, in der das Katheterspitzenelement (3, 3', 3", 3‴) zumindest abschnittsweise aus oder von der einführungsseitigen Öffnung (21, 21‴) in eine dem Katheterschlauch (2, 2‴) abgewandten Richtung vorsteht und eine geeignete Steifigkeit für eine Einführung des Katheters (1, 1', 1", 1‴) aufweist, in eine zweite Konfiguration, in der ein durch das vorstehende Katheterspitzenelement (3, 3', 3", 3‴) gebildeter Überstand des Katheterspitzenelements (3, 3', 3", 3‴) eine geringere Steifigkeit als in der ersten Konfiguration aufweist, überführbar ist,
wobei in der zweiten Konfiguration der durch das vorstehende Katheterspitzenelement (3, 3', 3", 3‴) gebildete Überstand des Katheterspitzenelements (3, 3', 3", 3‴) gegenüber der einführungsseitigen Öffnung (21, 21‴) zumindest teilweise aufgehoben ist,
wobei das Katheterspitzenelement (3, 3', 3", 3‴) zur Überführung von der ersten Konfiguration in die zweite Konfiguration in einer Erstreckungsrichtung des Katheterschlauchs (2, 2‴) von der einführungsseitigen Öffnung zu einem der einführungsseitigen Öffnung abgewandten Ende hin zumindest abschnittsweise relativ zum Katheterschlauch (2, 2‴) bewegbar ist, **dadurch gekennzeichnet, dass**
das Katheterspitzenelement (3, 3', 3") ein flexibles Rücknahmeelement (4, 4"), das sich ausgehend von dem Katheterspitzenelement (3, 3', 3") zumindest abschnittsweise durch den Katheterschlauch (2) erstreckt und zumindest in der Erstreckungsrichtung des Katheterschlauchs (2) relativ zu diesem bewegbar ist, umfasst.

2. Katheter (1, 1', 1", 1‴) nach Anspruch 1, wobei das Katheterspitzenelement (3) zumindest bei der Überführung von der ersten Konfiguration in die zweite Konfiguration einen maximalen Durchmesser (d2) senkrecht zur Erstreckungsrichtung des Katheterschlauchs (2, 2‴) aufweist, der kleiner oder gleich zumindest einem minimalen Innendurchmesser (d1) eines Rücknahmeabschnitts (22, 22‴) des Katheterschlauchs (2, 2‴) für das Katheterspitzenelement (3, 3', 3", 3‴) ist.

3. Katheter (1', 1", 1‴) nach Anspruch 1 oder 2, wobei zumindest der in der ersten Konfiguration aus und/oder von der einführungsseitigen Öffnung (21, 21‴) vorstehende Abschnitt des Katheterspitzenelements (3', 3", 3‴) zumindest einen verformbaren Abschnitt aufweist.

4. Katheter (1", 1‴) nach Anspruch 3, wobei zumindest der zumindest eine verformbare Abschnitt des Katheterspitzenelements (3", 3‴) so konfiguriert ist, dass es mit einem positiven und/oder negativen Innendruck beaufschlagbar ist.

5. Katheter (1‴) nach Anspruch 1 oder 2, wobei der in der ersten Konfiguration aus und/oder von der einführungsseitigen Öffnung (21‴) vorstehende Überstand des Katheterspitzenelements zumindest abschnittsweise in Richtung der Erstreckungsrichtung des Katheterschlauchs (2‴) nach innen, insbesondere in den Katheterschlauch (2‴) hinein, stülpbar ist.

6. Katheter (1, 1', 1") nach einem der vorherigen Ansprüche, wobei die einführungsseitige Öffnung des Katheterschlauchs (2) in der ersten Konfiguration durch das Katheterspitzenelement (3) fluiddicht abdichtbar ist.

7. Katheter (1) nach Anspruch 6, wobei die fluiddichte Abdichtung durch einen in Bezug auf die Erstreckungsrichtung des Katheterschlauchs (2) radial zumindest abschnittsweise umlaufenden Vorsprung (31) des Katheterspitzenelements (3) gebildet wird, der insbesondere zumindest in radialer Richtung komprimierbar ist.

8. Katheter (1, 1', 1", 1‴) nach einem der vorherigen Ansprüche, wobei der Katheterschlauch (2, 2‴) einen Katheteranker (23) aufweist, der in der Erstreckungsrichtung des Katheterschlauchs (2, 2‴) angrenzend an oder in einem vorbestimmten Abstand zur einführungsseitigen Öffnung (21, 21‴) angeordnet ist.

9. Katheter nach einem der vorherigen Ansprüche, wobei das Katheterspitzenelement eine Katheterspitzenelementöffnung aufweist, über die eine fluidische Verbindung zwischen einem die Katheterspitzenelementöffnung umgebenden Bereich, insbesondere einem Hohlorgan, und dem Katheterschlauch (2) ausbildbar ist.

10. Katheter nach einem der vorherigen Ansprüche, wobei der Katheterschlauch (2) neben der einführungsseitigen Öffnung zumindest eine zweite Öffnung aufweist, über die eine fluidische Verbindung zwischen einem die zweite Öffnung umgebenden Bereich, insbesondere einem Hohlorgan, und dem Katheterschlauch (2) ausbildbar ist.

11. Katheter (1, 1', 1", 1‴) nach einem der vorherigen Ansprüche, wobei das Katheterspitzenelement (3, 3', 3", 3‴) zumindest teilweise aus einem fluidlöslichen Material gebildet ist und/oder hiermit zumindest teilweise beschichtet ist und/oder mit einem Medikament beschichtet ist.

12. Katheter (1, 1', 1", 1 ‴) nach einem der vorherigen Ansprüche, wobei der Katheter (1, 1', 1", 1‴) ein Blasenkatheter, insbesondere ein transurethraler Blasenkatheter ist.

## Claims

1. A catheter (1, 1', 1", 1‴), comprising:
a flexible catheter tube (2, 2‴) with an insertion-side opening (21, 21‴) at an insertion-side end of the catheter tube (2, 2"'), and
a catheter tip element (3, 3', 3", 3‴), which is configured such that the catheter tip element (3, 3', 3", 3‴) is transferable from a first configuration, in which the catheter tip element (3, 3', 3", 3‴) projects at least in sections out of or from the insertion-side opening (21, 21‴) in a direction facing away from the catheter tube (2, 2‴) and has a suitable rigidity for an insertion of the catheter (1, 1', 1", 1‴), into a second configuration, in which a protrusion of the catheter tip element (3, 3', 3", 3‴) formed by the projecting catheter tip element (3, 3', 3", 3‴) has a lower rigidity than in the first configuration,
wherein in the second configuration the protrusion of the catheter tip element (3, 3', 3", 3‴) formed by the projecting catheter tip element (3, 3', 3", 3‴) is at least partially cancelled with respect to the insertion-side opening (21, 21‴),
wherein the catheter tip element (3, 3', 3", 3"') is movable at least in sections relative to the catheter tube (2, 2‴) in an extension direction of the catheter tube (2, 2‴) from the insertion-side opening towards an end facing away from the insertion-side opening for the transfer from the first configuration into the second configuration, **characterized in that**
the catheter tip element (3, 3', 3") comprises a flexible return element (4, 4"), which extends at least in sections through the catheter tube (2) starting from the catheter tip element (3, 3', 3") and movable at least in the extension direction of the catheter tube (2) relative thereto.

2. The catheter (1, 1', 1", 1‴) according to claim 1, wherein the catheter tip element (3) has a maximum diameter (d2) perpendicular to the extension direction of the catheter tube (2, 2‴) at least during the transfer from the first configuration into the second configuration, which is smaller than or equal to at least a minimum inner diameter (d1) of a return section (22, 22‴) of the catheter tube (2, 2"') for the catheter tip element (3, 3', 3", 3"').

3. The catheter (1', 1", 1‴ according to claim 1 or 2, wherein at least the section of the catheter tip element (3', 3", 3‴) projecting in the first configuration out of and/or from the insertion-side opening (21, 21‴) has at least one deformable section.

4. The catheter (1", 1‴ according to claim 3, wherein at least the at least one deformable section of the catheter tip element (3", 3‴) is configured such that it is capable of being subjected to a positive and/or negative internal pressure.

5. The catheter (1‴) according to claim 1 or 2, wherein the protrusion of the catheter tip element projecting in the first configuration out of and/or from the insertion-side opening (21‴) is capable of being pushed inwards at least in sections in the direction of the extension direction of the catheter tube (2‴), in particular into the catheter tube (2‴).

6. The catheter (1, 1', 1") according to any one of the preceding claims, wherein the insertion-side opening of the catheter tube (2) is sealable in a fluid-tight manner by the catheter tip element (3) in the first configuration.

7. The catheter (1) according to claim 6, wherein the fluid-tight seal is formed by a projection (31) of the catheter tip element (3), which projection is circumferential radially at least in sections with respect to the extension direction of the catheter tube (2) and is preferably compressible at least in the radial direction.

8. The catheter (1, 1', 1", 1‴ according to any one of the preceding claims, wherein the catheter tube (2, 2‴) has a catheter anchor (23) which is arranged adjacent to or at a predetermined distance from the insertion-side opening (21, 21‴) in the extension direction of the catheter tube (2, 2‴).

9. The catheter according to any one of the preceding claims, wherein the catheter tip element has a catheter tip element opening via which a fluidic connection is formable between a region surrounding the catheter tip element opening, in particular a hollow organ, and the catheter tube (2).

10. The catheter according to any one of the preceding claims, wherein the catheter tube (2) has, in addition to the insertion-side opening, at least one second opening via which a fluidic connection is formable between a region surrounding the second opening, in particular a hollow organ, and the catheter tube (2).

11. The catheter (1, 1', 1", 1‴ according to any one of the preceding claims, wherein the catheter tip element (3, 3', 3", 3‴) is at least partially formed from a fluid-soluble material and/or is at least partially coated therewith and/or is coated with a medicament.

12. The catheter (1, 1', 1", 1‴ according to any one of the preceding claims, wherein the catheter (1, 1', 1", 1‴ is a bladder catheter, in particular a transurethral bladder catheter.

## Revendications

1. Cathéter (1, 1', 1", 1‴), comprenant :
un tube de cathéter flexible (2, 2"') avec une ouverture côté introduction (21, 21‴) à une extrémité côté introduction du tube de cathéter (2, 2"'), et
un élément de pointe de cathéter (3, 3', 3", 3"'), qui est configuré de telle sorte que l'élément de pointe de cathéter (3, 3', 3", 3‴) peut être transféré d'une première configuration, dans laquelle l'élément de pointe de cathéter (3, 3', 3", 3"') fait saillie au moins en partie hors ou de l'ouverture côté introduction (21, 21‴) dans une direction opposée au tube de cathéter (2, 2‴) et présente une rigidité appropriée pour une introduction du cathéter (1, 1', 1", 1‴), à une deuxième configuration, dans laquelle un dépassement de l'élément de pointe de cathéter (3, 3', 3", 3"') formé par l'élément de pointe de cathéter faisant saillie (3, 3', 3", 3‴) présente une rigidité plus faible que dans la première configuration,
où, dans la deuxième configuration, le dépassement de l'élément de pointe de cathéter (3, 3', 3", 3‴) formé par l'élément de pointe de cathéter faisant saillie (3, 3', 3", 3‴) par rapport à l'ouverture côté introduction (21, 21‴) est au moins partiellement supprimé,
où, pour le transfert de la première configuration à la deuxième configuration, l'élément de pointe de cathéter (3, 3', 3", 3"') est déplacable au moins en partie par rapport au tube de cathéter (2, 2‴) dans une direction d'extension du tube de cathéter (2, 2"') de l'ouverture côté introduction vers une extrémité opposée à l'ouverture côté introduction,
**caractérisé en ce que** l'élément de pointe de cathéter (3, 3', 3") comprend un élément de retrait flexible (4, 4"), qui s'étend à partir de l'élément de pointe de cathéter (3, 3', 3") au moins en partie à travers le tube de cathéter (2) et est déplacable au moins dans la direction d'extension du tube de cathéter (2) par rapport à celui-ci.

2. Cathéter (1, 1', 1", 1‴) selon la revendication 1, où l'élément de pointe de cathéter (3) présente, au moins lors du transfert de la première configuration à la deuxième configuration, un diamètre maximal (d2) perpendiculaire à la direction d'extension du tube de cathéter (2, 2‴), qui est inférieur ou égal à au moins un diamètre intérieur minimal (d1) d'une section de retrait (22, 22‴) du tube de cathéter (2, 2"') pour l'élément de pointe de cathéter (3, 3', 3", 3‴).

3. Cathéter (1', 1", 1‴ selon la revendication 1 ou 2, où au moins la section de l'élément de pointe de cathéter (3', 3", 3‴) faisant saillie dans la première configuration hors et/ou de l'ouverture côté introduction (21, 21‴) présente au moins une section déformable.

4. Cathéter (1", 1‴ selon la revendication 3, où au moins l'au moins une section déformable de l'élément de pointe de cathéter (3", 3‴) est configurée de telle sorte qu'elle peut être soumise à une pression intérieure positive et/ou négative.

5. Cathéter (1‴) selon la revendication 1 ou 2, où le dépassement de l'élément de pointe de cathéter faisant saillie dans la première configuration hors et/ou de l'ouverture côté introduction (21‴) peut être retourné au moins en partie vers l'intérieur dans la direction de la direction d'extension du tube de cathéter (2‴), en particulier dans le tube de cathéter (2‴).

6. Cathéter (1, 1', 1") selon l'une des revendications précédentes, où l'ouverture côté introduction du tube de cathéter (2) peut être rendue étanche aux fluides dans la première configuration par l'élément de pointe de cathéter (3).

7. Cathéter (1) selon la revendication 6, où l'étanchéité étanche aux fluides est formée par une saillie (31) de l'élément de pointe de cathéter (3) s'étendant radialement au moins en partie par rapport à la direction d'extension du tube de cathéter (2), qui est en particulier compressible au moins dans la direction radiale.

8. Cathéter (1, 1', 1", 1‴) selon l'une des revendications précédentes, où le tube de cathéter (2, 2‴) présente un ancrage de cathéter (23) qui est disposé dans la direction d'extension du tube de cathéter (2, 2‴) adjacent à ou à une distance prédéterminée de l'ouverture côté introduction (21, 21‴).

9. Cathéter selon l'une des revendications précédentes, où l'élément de pointe de cathéter présente une ouverture d'élément de pointe de cathéter par laquelle une liaison fluidique peut être réalisée entre une zone entourant l'ouverture d'élément de pointe de cathéter, en particulier un organe creux, et le tube de cathéter (2).

10. Cathéter selon l'une des revendications précédentes, où le tube de cathéter (2) présente, outre l'ouverture côté introduction, au moins une deuxième ouverture par laquelle une liaison fluidique peut être réalisée entre une zone entourant la deuxième ouverture, en particulier un organe creux, et le tube de cathéter (2).

11. Cathéter (1, 1', 1", 1‴ selon l'une des revendications précédentes, où l'élément de pointe de cathéter (3, 3', 3", 3‴) est formé au moins partiellement d'un matériau soluble dans les fluides et/ou est au moins partiellement revêtu de celui-ci et/ou est revêtu d'un médicament.

12. Cathéter (1, 1', 1", 1‴ selon l'une des revendications précédentes, où le cathéter (1, 1', 1", 1‴ est un cathéter urinaire, en particulier un cathéter urinaire transurétral.
